# EUROPEAN PATENT SPECIFICATION

(11) **EP 3 017 880 B1**
(45) Date of publication and mention of the grant of the patent: **20.03.2019**
(21) Application number: 14192235.1
(22) Date of filing: 07.11.2014
(51) Int. Cl.: B08B 3/02, A47L 13/22, A47L 11/34, A47L 11/40, A47L 13/18

(54) **Steam cleaning device and accessory**
Dampfreinigungsvorrichtung und Zubehör
Dispositif et accessoire de nettoyage à la vapeur

(43) Date of publication of application: 11.05.2016
(73) Proprietor: Black & Decker Inc., Newark, Delaware 19711 (US)
(72) Inventor: Cooper, Vincent P., Shincliffe, Durham DH1 2ND (GB)
(74) Representative: SBD IPAdmin

(56) References cited:
- WO-A1-02/43550
- GB-A- 2 294 196
- DATABASE WPI Week 200818 Thomson Scientific, London, GB; AN 2008-C41865 XP002738494, -& JP 2008 011973 A (SHIMIZU CONSTR CO LTD) 24 January 2008 (2008-01-24)

## Description

The present invention relates to a steam cleaning device and accessory.

In recent times steam cleaning has become desirable in the domestic environment. A known steam cleaner is shown in EP2494901 which has a boiler for generating steam and a cleaning head for directing the steam to a surface to be cleaned. The cleaning head is designed to engage a floor surface. The size of the steam cleaner and the construction of the cleaning head means that it is difficult to clean surfaces other than the floor.

A cleaning device which is convenient to use for indoor domestic tasks is desirable. Embodiments of the present invention aim to address the aforementioned problems.

GB 2 294 196 discloses a mop head with means for applying steam to the surface to be cleaned. The head may be in the form of a round or flat frame connected to a handle. The head may be covered by a concertina or glove like mop or a nylon pad.

JP 2008 011973 discloses a steam cleaner with a pad. The pad has nozzles which jet steam to the surface. A handle for a worker to grasp is attached to the rear surface of the body.

WO 02/43550 discloses a cleaning glove comprising at least one duct coupled with the glove. The duct is suitable to allow suction from and or jets of fluids outside the holes.

According to an aspect of the present invention there is a wearable steam cleaning accessory according to claim 1.

The steam cleaning accessory is conveniently wearable on the user's hand. Furthermore the steam cleaning accessory comprises a plurality of layers, each of which is flexible and one of the layers delivers and outputs steam. This makes a convenient a deformable steam cleaning accessory which outputs steam at a temperature which kills germs. In particular the steam cleaning accessory is convenient for sanitizing non-flat surfaces such as toilets, taps, shower heads and sinks.

In comparison one known hand held cleaning accessory is shown in DE9314368 U1 which comprises a washing glove for cleaning cars and animals.

However this washing glove is only suitable for use with water. The washing glove has an upper part for receiving the user's hand and a wash pad receives water from a hose connection. The problem with this cleaning accessory is that it is only suitable for outdoor use where large quantities of waste water can easily be managed.

However many indoor domestic cleaning tasks cannot be carried out with a large flow of running water because the water will cause damage or create more mess. For example the water may drip onto other surfaces when wiping vertical surfaces such as cupboard doors or and the water may fill up a refrigerator drip tray when cleaning the inside of a refrigerator. In contrast the steam cleaning accessory of the present invention is able to clean and sanitize surfaces without the concern that water will drip off a vertical surface or interfere with the operation of a refrigerator.

Preferably the at least one flexible thermal insulation layer comprises at least one air inlet and at least one air outlet and an air flow path therebetween. This increases the convection of the surrounding air and increases the cooling of the steam cleaning accessory. This means that the user can comfortably hold the steam cleaning accessory for an extended period of time.

Preferably one of the at least one air inlet or the at least one air outlets are located on a peripheral side of the steam cleaning accessory. This means that increased air circulation is achieved because the warm air in the thermal insulation layer is replaced with cooler surrounding air each time the steam cleaning accessory is moved from side to side. In this way the wiping action a user carries out when cleaning a surface will create an improved cooling effect with the increased convection of air through the thermal insulation layer.

Preferably there are a plurality of air inlets and air outlets for even more increased air circulation in the flexible thermal insulation layer.

Preferably the at least one flexible thermal insulation layer comprises a resilient material arranged to return to a predetermined shape after being deformed. Preferably wherein the spacer fabric comprises a first fabric layer and a second fabric layer and the first and second fabric layers are separated by resilient flexible threads knitted between the first and second layers. This means that the resilient material can keep the air inlet and air outlet open even when the steam cleaning accessory is being deformed and flexed.

Preferably the at least one flexible thermal layer comprises a first flexible thermal insulation layer and a second flexible thermal insulation layer. Preferably the second flexible thermal insulation layer is separable from the first flexible thermal insulation layer. This means that the second and first thermal insulation materials can be separated to be cleaned and the steam ducts do not have to be washed at the same time as the first thermal insulation material.

Preferably the at least one steam conduit is bonded to the second flexible thermal insulation layer.

Preferably the flexible cleaning element is one or more of the following a cleaning cloth, a scourer, bristles, or a sponge. Preferably the flexible cleaning element is removeably mounted to the at least one flexible thermal insulation layer. This means that the flexible cleaning element can be separately cleaned from the rest of the steam cleaning accessory. In addition the flexible cleaning element can be replaced when necessary.

Preferably the at least one steam conduit comprises a flexible tube with a D shaped cross section. The flat side of the D shaped cross section can face the thermal insulation layer and the flat side limits the rotation of the steam conduit during manufacture. This helps to make sure the steam outlets which may be pre-cut in the material of the steam conduit face the correct way after the steam conduit has been bonded to the thermal insulation layer.

Preferably the at least one steam outlet is mounted on a surface of the at least one steam conduit which faces the flexible cleaning element. In this way the steam outlets face towards the flexible cleaning element. This ensures the steam flows out of the steam cleaning accessory and not inwards towards the user's hand.

Preferably the restraint comprises a pocket having a mesh material for circulating air near the user's hand. The mesh material allows air circulation around the user's hand which increases convection of the warm air and keeps the user's hand cool.

Preferably the at least one flexible thermal insulation layer comprises a peripheral wall projecting towards the flexible restraint for substantially enclosing the user's hand. The peripheral wall provides a structure against which the users hand can abut when the steam cleaning accessory is being used. The peripheral wall ensures that the user's hand remains in the same position with respect to the steam cleaning accessory during use.

In another aspect of the invention there is a steam cleaning device comprising; a steam cleaning accessory according to the aforementioned aspects of the invention; and a steam generating device coupled to the adaptor.

Various other aspects and further embodiments are also described in the following detailed description and in the attached claims with reference to the accompanying drawings, in which:
Figure 1 shows a schematic representation of the steam cleaning device and accessory according to an embodiment;
Figure 2 shows a cross sectional side view of the steam cleaning accessory according to an embodiment;
Figure 3 shows a schematic representation of a partial view of the steam cleaning accessory.
Figure 4 shows a cross sectional side view of the steam cleaning accessory according to an embodiment;
Figure 5 shows a cross sectional side view of the steam cleaning accessory according to an embodiment;
Figure 6 shows a cross sectional front view of the steam cleaning accessory according to an embodiment;
Figure 7 shows a cross sectional front view of the steam cleaning accessory according to an embodiment;
Figure 8 shows a cross sectional front view of the steam cleaning accessory according to an embodiment; and
Figure 9 shows a picture of the steam cleaning device and accessory in use.

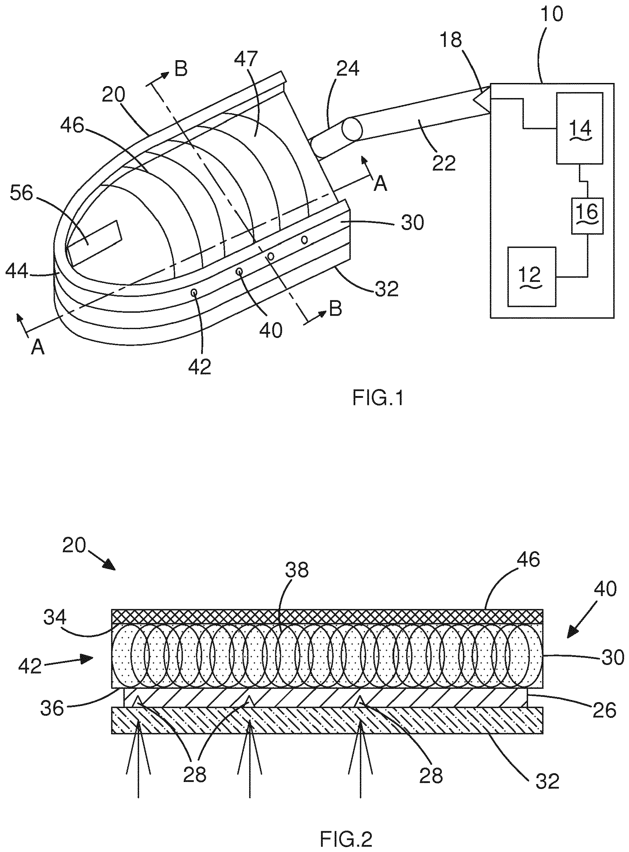

Figure 1 shows a schematic perspective view of a steam cleaning device 10. The steam cleaning device 10 comprises a water tank 12 and a steam generator such as a boiler 14. A pump 16 pumps water to the boiler 14. The boiler 14 comprises a resistive element and is powered by a source of electrical energy such as mains electricity or battery. Steam is generated by the boiler 14 and output at a steam nozzle 18 (or any other suitable steam outlet of the steam cleaning device 10).

The steam cleaning device 10 is coupled to a steam cleaning accessory 20 by a steam hose 22 and an adaptor 24. The adaptor 24 which is mounted on the steam cleaning accessory 20 and is arranged to couple the steam cleaning accessory 20 with the steam nozzle 18 such that the steam cleaning device 10 is in fluid communication with the steam cleaning accessory 20 via the steam hose 22.

The steam cleaning device 10 comprises a coupling for fixing the steam hose 22 to the steam nozzle 18. The steam hose 22 is detachable from the adaptor 24 allowing the steam cleaning device 10 to be used with other steam cleaning accessories. The steam cleaning device 10 is handheld and the steam cleaning accessory 20 is wearable on the other hand of the user. Of course, the user can also wear the steam cleaning accessory 20 without holding the steam cleaning device 10 at the same time. The steam hose 22 is of sufficient length such that the steam cleaning accessory 20 can be moved without constantly moving the steam cleaning device 10.

For example, in some embodiments the steam hose is about 50cm to 100cm in length. This means that steam hose 22 is about the same length as a user's arm and the user can move the steam cleaning accessory 20 without moving the steam cleaning device 10 when held in the other hand.

Although not shown in Figure 1, in some other embodiments the steam hose 22 is fixed to the steam cleaning accessory 20 and the adaptor 24 is mounted at the end of the steam hose 22. When the adaptor 24 is mounted at the end of the steam hose 22, the adaptor 24 couples the steam hose 22 to the steam nozzle 18.

The steam cleaning accessory 20 will now be described in more detail with reference to figures 2 to 8. Figure 2 shows a side cross sectional view of the steam cleaning accessory 20 along the line A-A in Figure 1. The steam cleaning accessory 20 comprises a steam conduit or steam duct 26. The steam duct 26 is in fluid communication with the adaptor 24 and the steam cleaning device 10. The steam duct 26 in some embodiments is a silicone tube which extends over the steam cleaning accessory 20. The steam duct comprises one or more steam outlets 28 for allowing steam to be released from the steam duct 26.

The steam duct 26 is mounted between a flexible thermal insulation layer 30 and a flexible cleaning element 32. Although not shown, the flexible cleaning element 32 is also fixed to the flexible thermal insulation layer 30. The flexible cleaning element 32 is steam permeable and the steam outlets 28 face the flexible cleaning element 32. The flexible cleaning element 32 is steam permeable by virtue that the flexible cleaning element is a material which comprises holes for allowing steam to pass therethrough. In some embodiments the flexible cleaning element 32 is a fabric material such as a woven fabric material. The woven fabric material has holes between the threads and the holes allow the steam to pass through. Alternatively the flexible cleaning element 32 is a substantially non steam permeable material, but the flexible cleaning element 32 comprises through holes for allowing the passage of steam through the flexible cleaning element.

In some embodiments the flexible cleaning element 32 is a cleaning cloth which is suitable for wiping along dirty surfaces. In other embodiments the flexible cleaning element 32 can be one or more of the following, a cleaning element with bristles, brush, a scourer, sponge, pad or any suitable material for cleaning and wiping a dirty surface. Since the flexible cleaning element 32 is deformable, the flexible cleaning element can be wrapped around curved surfaces such as taps, sinks and the like.

As mentioned above, the steam duct 26 is sandwiched between the flexible cleaning element 32 and the flexible thermal insulation layer 30. The flexible thermal insulation layer 30 is a barrier layer which limits the transmission of the thermal energy across the steam cleaning accessory 20. The flexible thermal insulation layer 30 can be any suitable thermal insulation layer which is flexible. For example in some embodiments the flexible thermal insulation layer is a silicone layer.

In some alternative embodiments, and as shown in Figure 2, the flexible thermal insulation layer 30 is a spacer fabric. The spacer fabric comprises a first layer 34 of fabric and a second layer 36 of fabric and the first and second layers are separated by at least one resilient thread 38 knitted therebetween. This means that the spacer fabric comprises an air inlet 40 and an air outlet 42 and an air flow pathway between the two. The air inlet 40 and the air outlet 42 can be located in any position on the flexible thermal layer 30 and there may be any number of air inlets 40 or air outlets 42. This means that the convection of air is increased around and through the flexible thermal insulation layer 30. In some embodiments the first layer 34 and the second layer 36 of the spacer fabric are a mesh or net like material and comprise a plurality of holes which promote air flow within the flexible thermal insulation layer 30.

The inventor has realised that a flexible thermal insulation layer 30 with at least one an air inlet 40 and an air outlet 42 with an air flow pathway between them is an effective way of preventing thermal energy building up in the steam cleaning accessory 20 from the continual use of the steam cleaning accessory The embodiments discussed herein dissipate the thermal energy from the steam cleaning accessory 20 by convection of the surrounding air through the steam cleaning accessory.

In some other embodiments the flexible thermal insulation layer 30 comprises a foam material which comprises holes allowing air to pass therethrough. In other embodiments the thermal insulation layer 30 is a solid material such as a silicone layer with holes bored into the centre of the material for allowing air to circulate through the centre of the silicone layer.

Briefly turning to Figure 1, the flexible thermal insulation layer 30 optionally comprises at least one of the air inlet 40 and the air outlet 42 in a peripheral side 44 of the steam cleaning accessory. By having air holes in the peripheral side 44 of the steam cleaning accessory 20, when the steam cleaning accessory 20 is moved from side to side, air from the external environment is encouraged to move along the air flow pathway. This means cool air from outside the steam cleaning accessory 20 replaces the warmer air within the flexible thermal insulation layer 30 each time the steam cleaning accessory 20 is moved from side to side. The air inlet 40 and the air outlet 42 can optionally be in alternative positions around the thermal insulating later 30.

A flexible retaining layer 46 is mounted on the flexible thermal insulation layer 30. The flexible retaining layer 46 is fixed to the side of the flexible thermal insulation layer 30 which is opposite to the side on which the steam duct 26 is mounted. The flexible retaining layer 46 in some embodiments is a flexible restraint for receiving the user's hand. The flexible restraint in some embodiments can be a flexible pocket 47. The flexible layer 46 creates a flexible pocket 47 between the retaining layer 46 and the flexible thermal insulation layer 30 in which the user can place their hand. When the user puts their hand in the flexible pocket 47, the flexible thermal insulation layer 30 and the retaining layer 46 deform around the hand. In this way the user is able to wear the steam cleaning accessory 20 in the same way they can wear a glove or a mitt. In use the user's palm is adjacent to the first fabric layer 34 of the flexible thermal insulation layer 30 and the back of the user's hand is adjacent to the flexible retaining layer 46. The flexible retaining layer 46 and the flexible pocket 47 allow the steam cleaning accessory 20 to be worn on the hand without physically gripping the steam cleaning accessory 20. This means the steam cleaning accessory 20 does not fall off the user's hand.

Optionally in some embodiments the retaining layer 46 is a mesh material or a net material. This provides air holes in the retaining layer 46 and increases the circulation of air around the user's hand which helps keep the user's hand cool.

In some embodiments the retaining layer 46 comprises an elasticated material which further grips the user's hand. The retaining layer 46 may also comprise one or more upstanding finger partitions 56 for separating a user's fingers. The finger partitions 56 aid the user's comfort when using the steam cleaning accessory. Optionally the retaining layer 46 may comprise a releasable cuff for wrapping around the user's wrist to help keep the steam cleaning accessory on the user's hand.

The flexible restraint can be any suitable means for coupling the user's hand to the steam cleaning accessory 20. Alternatively the flexible restraint is one or more flexible straps which are mounted to the flexible thermal insulation layer 30. The flexible straps (not shown) pass over the back of the user's hand and/ or wrist.

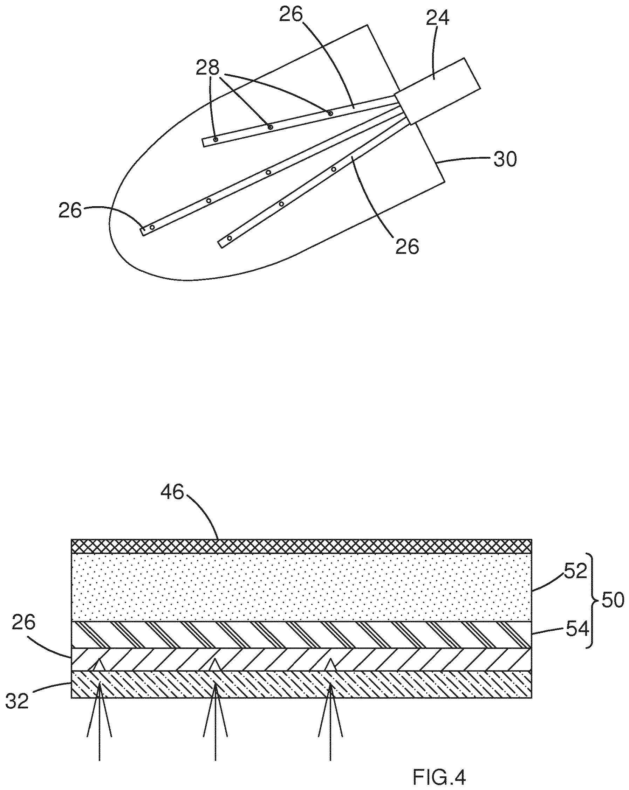

The distribution of the steam duct 26 will now be discussed in further detail to Figure 3. Figure 3 shows an underneath plan view of part of the steam cleaning accessory 20. In particular figure 3 shows three steam ducts 26 mounted on the flexible thermal insulating layer 30. Each steam duct is in fluid communication with the adaptor 24 and the steam cleaning device 10. The plurality of steam ducts 26 each comprises at least one steam outlet 28. Figure 3 shows that each steam duct 26 has a plurality of steam outlets 28. The steam cleaning accessory 20 can have any number of steam ducts 26 and the steam ducts 26 can follow any path over the flexible thermal insulation layer 30.

Further embodiments will now be discussed in reference to Figure 4. Figure 4 shows a cross sectional side view of the steam cleaning accessory 20. The steam cleaning accessory 20 is similar to the embodiments discussed in reference to Figures 1 to 3. The same reference numbers will be used for the same features in previously mentioned embodiments. Figure 4 differs in that the flexible thermal insulation layer 50 comprises a first flexible thermal insulation layer 52 and a second flexible thermal insulation layer 54. The first flexible thermal insulation layer 52 is the same as the flexible thermal insulation layer 30 in the embodiments described with respect to Figures 1 to 3. The second flexible thermal insulation layer 54 is a solid flexible layer on which the steam duct 26 is mounted. By separating the flexible thermal insulation layer 50 in to two different parts, a thinner composite material can be achieved. The solid flexible layer 54 is non-woven and reduces the amount of heat radiated from the steam ducts 26 to the user's hand. The second flexible layer can be a flexible layer of silicone. The first flexible thermal insulation layer 52 is thinner compared to the thermal insulation layer 30 in the embodiment discussed in Figure 3. The boundary layer between the first and second layers 52, 54 also reduces the amount of thermal energy conducted through the materials.

By providing a silicone layer 54 or another non-woven thermally insulating material, the steam duct 26 is more easily bonded and fixed in place. In some embodiments the silicone tubes used for the steam duct 26 are bonded to the silicone layer 54 with a silicone based adhesive. In some other embodiments the silicone tube 26 and the silicone layer 54 are partially cured. During manufacture the partially cured silicone tubes 26 are placed in position on the partially cured silicone layer 54 and the arrangement is exposed to an elevated temperature. This cures both the silicone tube 26 to the silicone layer 54 which are both bonded to each other without the need for adhesive.

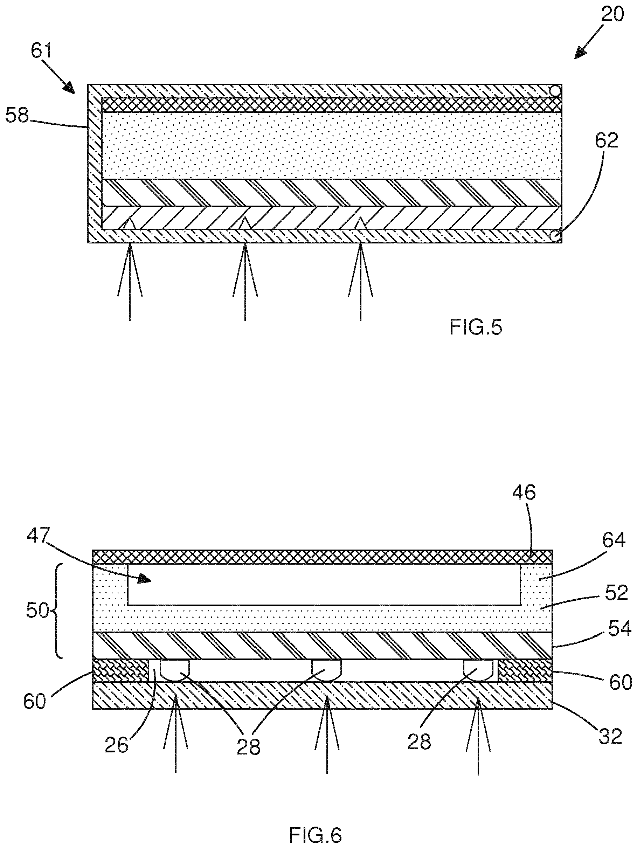

Turning to Figure 5 another embodiment of the steam cleaning accessory 20 will now be discussed. The steam cleaning accessory 20 is similar to the embodiment discussed with reference to the embodiments shown in Figure 4 and the same reference numbers will be used to indicate the same features. Figure 5 differs in that the flexible cleaning element 32 is removeable and replaceable. The flexible cleaning element as shown in Figure 5 is a replaceable cleaning sock 58 which covers the entire steam cleaning accessory 20. The cleaning sock 58 is made from the same material as the flexible cleaning element 32 as discussed in reference to the embodiments of Figures 1 to 4. The opening of the cleaning sock 58 has an elasticated band 62 or a draw string for fastening the cleaning sock 58 to the steam cleaning accessory 20. In some alternative embodiments, the cleaning sock 58 has a pocket portion (not shown) in which the finger end 61 of the steam cleaning accessory 20 is inserted and the cleaning sock 58 is fastened to the steam cleaning accessory 20 at the other end. The replaceable cleaning sock 58 can be used in conjunction with any of the other embodiments discussed herein.

Figure 6 shows a front cross sectional view of the steam cleaning accessory 20 as view along cross section B-B. The steam cleaning accessory 20 is the same as the steam cleaning accessory 20 as shown in Figure 4 and the same reference numbers will be used accordingly. The flexible cleaning element 32 is removeably mounted on the second flexible thermal insulation layer 54. The flexible cleaning element 32 is fastened to the second flexible thermal insulation layer with a hook and eye arrangement 60 (e.g. VELCRO®). Alternatively any suitable fastening means can be used to removeably fasten the cleaning element 32 to the thermal insulation layer 54. For example clips or screws could be used instead. Removeably attaching the flexible cleaning element 32 to the thermal insulation layer 50 may be optionally used in conjunction with any of the other embodiments discussed herein.

The steam ducts 26 are mounted on the second flexible thermal insulation layer 54. The steam ducts 26 project down from the second flexible thermal insulation layer 54. Flexible infill material (not shown) may be located between the steam ducts 26 so that the flexible cleaning element 32 e.g. a cloth does not wrinkle or crease around the steam ducts 26. Optionally the steam ducts 26 have a "D-shaped" cross section with the flat side adjacent to the second flexible thermal insulation layer 54. The flat surface of the steam duct allows the steam ducts 26 and the steam outlets 28 to be aligned before bonding to the insulation layer 54. This means that the steam outlets 28 are less likely to be pointing in the wrong direction, for example towards the user's hand because the flat surface limits rotation of the steam duct 26 during manufacture.

The first layer of the flexible thermal insulating layer 52 may optionally comprise an upstanding peripheral wall 64. The upstanding peripheral wall 64 substantially encircles the users hand. This means that the peripheral wall 64 defines an interior recess which increases the size of the pocket 47. The peripheral wall 64 also helps the user's hand remain engage with the steam cleaning accessory 20 in a central position when wiping surfaces. In other words the peripheral wall 64 gives the user something to push against when wiping the steam cleaning accessory from side to side.

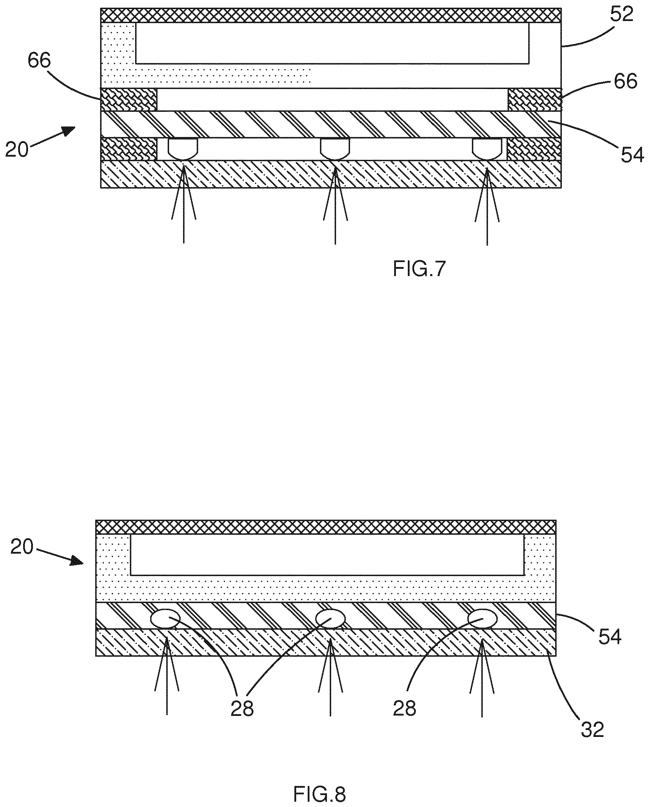

Figure 7 shows a front cross sectional view of another embodiment of the steam cleaning accessory. Figure 7 shows a similar steam cleaning accessory 20 as shown in Figure 6. Figure 7 differs from Figure 6 in that the second flexible thermal insulation layer 54 is removeable from the first flexible thermal insulation layer 52. The first and second layers 52, 54 are coupled to each other by a hook and eye fastening arrangement 66 (e.g. VELCRO®). Any other suitable fastening means can be used to fasten the first and second flexible thermal insulation layers together 52, 54. By making the first and second layers flexible thermal insulation 52, 54 separable, the first flexible thermal insulation layer 52 can be washed independently of the steam ducts. Removeably attaching the first and second thermal insulation layers 52, 54 may be optionally used in conjunction with any of the other embodiments discussed herein.

Another embodiment of the steam cleaning accessory 20 will now be discussed in reference to Figure 8. Figure 8 shows a front cross sectional view of the steam cleaning accessory. The steam cleaning accessory 20 of Figure 8 is similar to the steam cleaning accessory 20 described with reference to the previous embodiments. The difference is that the steam ducts 26 are embedded or partially embedded in the second flexible thermal insulation layer 54. This means that the flexible cleaning element 32 sits flush on the thermal insulation layer 54. Alternatively the steam ducts 26 may comprise integral bores completely within the second flexible thermal insulation layer 54 for providing a flow pathway for the steam.

In another embodiment, not shown, the steam conduit is a bladder formed from two pieces of steam impermeable material bonded together. The bladder comprises a plurality of holes for releasing the steam towards the flexible cleaning element 32, similar to the previously discussed embodiments. The steam fills up the bladder and creates a steam reservoir within the steam cleaning accessory 20. In some embodiments the bladder can also form the second flexible thermal insulation layer. Alternatively, the bladder is formed from a single piece of material having a balloon-like construction.

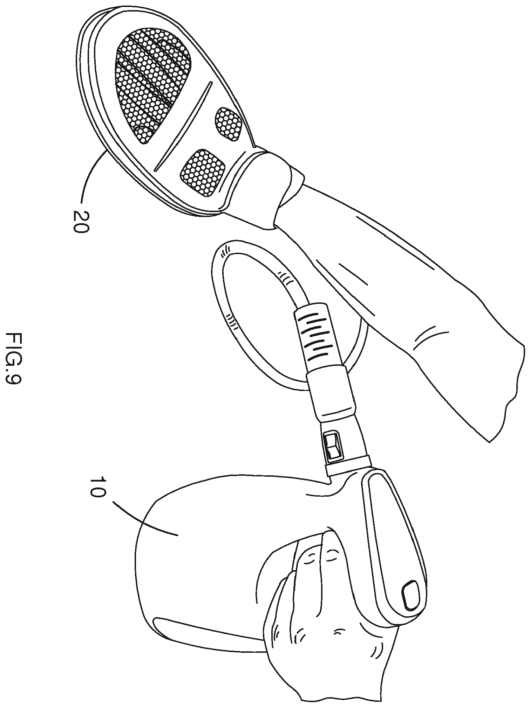

Use of the steam cleaning accessory 20 will now discussed in reference to Figure 9. Figure 9 shows a photo of the steam cleaning device 10 which is held in the hand and the steam cleaning accessory 20 worn on the other hand. The steam cleaning device generates steam and this flows through the steam ducts 26 and out of the steam outlets 28. The flexible thermal insulation layer 30 stops the users hand getting hot or burnt. Since the entire steam accessory 20 is flexible, the steam accessory 20 can be deformed, bent and moulded according to the position of the users hand. The steam cleaning accessory will deform and bend around curved surfaces allowing the user to achieve a steam clean. This is particularly advantageous when cleaning toilets, showerheads, taps and sinks.

The steam cleaning device 10 may comprise a small boiler 14 which delivers between 5ml/min to 30ml/min of steam to the steam cleaning accessory 20. In some embodiments the boiler generates 15 - 20ml/min of steam. It is thought that 15-20ml/min of steam will provide enough steam to the steam cleaning accessory 20 to achieve germ kill.

In some alternative embodiments (not shown) the steam cleaning accessory 20 comprises a first portion for one or more digits and a second portion for one or more digits. The first and second portions are independently moveable with respect to each other. The first and second portions comprises a split therebetween which provides a receiving space. Each layer comprises the first and second portions such that the first and second portions each respectively operates as a steam cleaning accessory. The first and second portions may each comprise a steam duct 26. Alternatively the steam duct 26 may optionally not extend into the first and second portion, but only extend into an area adjacent to the user's palm.

The receiving space is configured to accommodate a surface to be cleaned. In some embodiments the first portion is a thumb portion for receiving the thumb and the second portion is a finger portion for receiving one or more fingers. The thumb portion is spaced apart from the finger portion due to the natural hand shape. The receiving space is located between the thumb portion and the finger portion and is suitable for wrapping around pipes or other elongate objects. This means steam cleaning can be achieved on a round pipe more easily. In a further embodiment there is a plurality of splits in the steam cleaning accessory. This means that the steam cleaning accessory 20 can be a glove having from three to five separate portions, each configured to operate as a steam cleaning accessory 20.

In another embodiment (not shown), the steam cleaning accessory 20 comprises a flexible restraint for receiving less than five digits of a user's hand. For example the flexible restraint is sized only to receive two fingers (e.g. the index and the middle fingers). In other respects, the steam cleaning accessory 20 is the same as the steam cleaning accessories 20 as described in reference to the previously discussed embodiments. This means that that a flexible restraint only receiving two fingers can be smaller and this means the steam cleaning accessory 20 allows more detailed and precise cleaning.

In another embodiment two or more embodiments are combined. Features of one embodiment can be combined with features of other embodiments.

Embodiments of the present invention have been discussed with particular reference to the examples illustrated. However it will be appreciated that variations and modifications may be made to the examples described within the scope of the claims.

## Claims

1. A wearable steam cleaning accessory (20) for use with a steam generator and wearable on a user's hand comprising:
an adaptor (24) for coupling to the steam generator (14);
at least one steam conduit (26) in fluid communication between the adaptor (24) and at least one steam outlet (26);
a flexible cleaning element (32) for engaging with a surface, wherein the cleaning element is steam permeable;
at least one flexible thermal insulation layer (30,50) wherein the at least one steam conduit (26) is mounted between the at least one flexible thermal insulation layer (30) and the flexible cleaning element (32); and
a flexible restraint (46) mounted on the flexible thermal insulation layer (30,50) and the flexible restraint (46) is arranged to receive the user's hand.

2. A wearable steam cleaning accessory according to claim 1 wherein the at least one flexible thermal insulation layer (30) comprises at least one air inlet (40) and at least one air outlet (42) and an air flow path therebetween.

3. A wearable steam cleaning accessory according to claim 2 wherein one of the at least one air inlet (40) or the at least one air outlet (42) are located on a peripheral side (44) of the steam cleaning accessory.

4. A wearable steam cleaning accessory according to claims 1 to 3 wherein the at least one flexible thermal insulation layer (30) comprises a resilient material arranged to return to a predetermined shape after being deformed.

5. A wearable steam cleaning accessory according to claim 4 wherein the resilient material comprises a first fabric layer (34) and a second fabric layer (36) and the first and second fabric layers are separated by resilient flexible threads knitted between the first and second fabric layers.

6. A wearable steam cleaning accessory according to any of claims 1 to 5 wherein the at least one flexible thermal layer (50) comprises a first flexible thermal insulation layer (52) and a second flexible thermal insulation layer (54).

7. A wearable steam cleaning accessory according to any of claim 6 wherein the second flexible thermal insulation layer (54) is separable from the first flexible thermal insulation layer (52).

8. A wearable steam cleaning accessory according to any of the claims 6 and 7 wherein the at least one steam conduit (26) is bonded to the second flexible thermal insulation layer (54).

9. A wearable steam cleaning accessory according to any of the preceding claims wherein the flexible cleaning element (32) is one or more of the following a cleaning cloth, a scourer, bristles, or a sponge.

10. A wearable steam cleaning accessory according to any of the preceding claims wherein the flexible cleaning element (32) is removeably mounted to the at least one flexible thermal insulation layer (30, 50).

11. A wearable steam cleaning accessory according to any of the preceding claims wherein the at least one steam conduit (26) comprises a flexible tube with a D shaped cross section.

12. A wearable steam cleaning accessory according to any of the preceding claims wherein the at least one steam outlet (26) is mounted on a surface of the at least one steam conduit which faces the flexible cleaning element (32).

13. A wearable steam cleaning accessory according to any of the preceding claims wherein the restraint (46) comprises a pocket having a mesh material for circulating air near the user's hand.

14. A wearable steam cleaning accessory according to any of the preceding claims wherein the at least one flexible thermal insulation layer (30, 50) comprises a peripheral wall projecting towards the flexible restraint for substantially enclosing the user's hand.

15. A steam cleaning device (10) comprising;
a wearable steam cleaning accessory (20) according to claims 1 to 14; and
a steam generating device (14) coupled to the adaptor.

## Patentansprüche

1. Tragbares Dampfreinigungszubehör (20) zur Verwendung mit einem Dampfgenerator und tragbar auf einer Hand eines Benutzers, umfassend:
einen Adapter (24) zum Koppeln mit dem Dampfgenerator (14);
mindestens eine Dampfleitung (26) in Fluidkommunikation zwischen dem Adapter (24) und mindestens einem Dampfelement (26);
ein flexibles Reinigungselement (32) zum Eingriff mit einer Oberfläche, wobei das Reinigungselement dampfdurchlässig ist;
mindestens eine flexible thermische Isolationsschicht (30, 50), wobei die mindestens eine Dampfleitung (26) zwischen der mindestens einen flexiblen thermischen Isolationsschicht (30) und dem flexiblen Reinigungselement (32) montiert ist; und
eine flexible Halterung (46), montiert auf der flexiblen Isolationsschicht (30, 50) und wobei die flexible Halterung (46) angeordnet ist, um die Hand des Benutzers zu empfangen.

2. Tragbares Dampfreinigungszubehör nach Anspruch 1, wobei die mindestens eine flexible thermische Isolationsschicht (30) mindestens einen Lufteinlass (40) und mindestens einen Luftauslass (42) und einen Luftflusspfad dazwischen umfasst.

3. Tragbares Dampfreinigungszubehör nach Anspruch 2, wobei einer des mindestens einen Lufteinlasses (40) oder der mindestens eine Luftauslass (42) auf einer peripheren Seite (44) des Dampfreinigungszubehörs lokalisiert sind.

4. Tragbares Dampfreinigungszubehör nach einem der Ansprüche 1 bis 3, wobei die mindestens eine flexible thermische Isolationsschicht (30) ein elastisches Material umfasst, angeordnet, um in eine vorbestimmte Gestalt zurückzukehren, nachdem es deformiert wurde.

5. Tragbares Dampfreinigungszubehör nach Anspruch 4, wobei das elastische Material eine erste Gewebeschicht (34) und eine zweite Gewebeschicht (36) umfasst und wobei die ersten und zweiten Gewebeschichten mittels elastischer flexibler Fäden separiert sind, die zwischen den ersten und zweiten Gewebeschichten gestrickt sind.

6. Tragbares Dampfreinigungszubehör nach einem der Ansprüche 1 bis 5, wobei die mindestens eine flexible thermische Schicht (50) eine erste flexible thermische Isolationsschicht (52) und eine zweite flexible thermische Isolationsschicht (54) umfasst.

7. Tragbares Dampfreinigungszubehör nach einem der Ansprüche 6, wobei die zweite flexible thermische Isolationsschicht (54) von der ersten flexiblen thermischen Isolationsschicht (52) separiert ist.

8. Tragbares Dampfreinigungszubehör nach einem der Ansprüche 6 und 7, wobei die mindestens eine Dampfleitung (26) mit der zweiten flexiblen thermischen Isolationsschicht (54) gebunden ist.

9. Tragbares Dampfreinigungszubehör nach einem der vorstehenden Ansprüche, wobei das flexible Reinigungselement (32) eines oder mehrere der folgenden eines Reinigungstuchs, eines Scheuerlappens, Borsten oder eines Schwammes ist.

10. Tragbares Dampfreinigungszubehör nach einem der vorstehenden Ansprüche, wobei das flexible Reinigungselement (32) entfernbar auf der mindestens einen flexiblen thermischen Isolationsschicht (30, 50) montiert ist.

11. Tragbares Dampfreinigungszubehör nach einem der vorstehenden Ansprüche, wobei die mindestens eine Dampfleitung (26) ein flexibles Rohr mit einem D-förmigen Querschnitt umfasst.

12. Tragbares Dampfreinigungszubehör nach einem der vorstehenden Ansprüche, wobei der mindestens eine Dampfauslass (26) auf einer Oberfläche der mindestens einen Dampfleitung montiert ist, die dem flexiblen Reinigungselement (32) gegenüber liegt.

13. Tragbares Dampfreinigungszubehör nach einem der vorstehenden Ansprüche, wobei die Halterung (46) eine Tasche umfasst, die ein Netzmaterial zum Zirkulieren von Luft nahe der Hand des Benutzers hat.

14. Tragbares Dampfreinigungszubehör nach einem der vorstehenden Ansprüche, wobei die mindestens eine flexible thermische Isolationsschicht (30, 50) eine periphere Wand umfasst, die in Richtung auf die flexible Halterung vorspringt, um die Hand des Benutzers im Wesentlichen zu umschließen.

15. Dampfreinigungsvorrichtung (10), umfassend:
ein tragbares Dampfreinigungszubehör (20) nach Ansprüchen 1 bis 14; und
eine Dampferzeugungsvorrichtung (14), gekoppelt mit dem Adapter.

## Revendications

1. Accessoire de nettoyage à la vapeur prêt-à-porter (20) pour une utilisation avec un générateur de vapeur et pouvant être porté sur la main d'un utilisateur comprenant :
un adaptateur (24) destiné à être couplé au générateur de vapeur (14) ;
au moins un conduit de vapeur (26) en communication fluidique entre l'adaptateur (24) et au moins une sortie de vapeur (26) ;
un élément de nettoyage souple (32) destiné à venir en prise avec une surface, dans lequel l'élément de nettoyage est perméable à la vapeur ;
au moins une couche d'isolation thermique souple (30, 50) dans lequel l'au moins un conduit de vapeur (26) est monté entre l'au moins une couche d'isolation thermique souple (30) et l'élément de nettoyage souple (32) ; et
un dispositif de retenue souple (46) monté sur la couche d'isolation thermique souple (30,50) et le dispositif de retenue souple (46) est agencé pour recevoir la main d'un utilisateur.

2. Accessoire de nettoyage à la vapeur prêt-à-porter selon la revendication 1 dans lequel l'au moins une couche d'isolation thermique souple (30) comprend au moins une entrée d'air (40) et au moins une sortie d'air (42) et un trajet d'écoulement d'air entre celles-ci.

3. Accessoire de nettoyage à la vapeur prêt-à-porter selon la revendication 2 dans lequel l'une de l'au moins une entrée d'air (40) ou de l'au moins une sortie d'air (42) est située sur un côté périphérique (44) de l'accessoire de nettoyage à la vapeur.

4. Accessoire de nettoyage à la vapeur prêt-à-porter selon les revendications 1 à 3 dans lequel l'au moins une couche d'isolation thermique souple (30) comprend un matériau élastique agencé pour reprendre une forme prédéterminée après avoir été déformé.

5. Accessoire de nettoyage à la vapeur prêt-à-porter selon la revendication 4 dans lequel le matériau élastique comprend une première couche de tissu (34) et une seconde couche de tissu (36) et les première et seconde couches de tissu sont séparées par des fils souples élastiques tricotés entre les première et seconde couches de tissu.

6. Accessoire de nettoyage à la vapeur prêt-à-porter selon l'une quelconque des revendications 1 à 5 dans lequel l'au moins une couche thermique souple (50) comprend une première couche d'isolation thermique souple (52) et une seconde couche d'isolation thermique souple (54).

7. Accessoire de nettoyage à la vapeur prêt-à-porter selon l'une quelconque de la revendication 6 dans lequel la seconde couche d'isolation thermique souple (54) est séparable de la première couche d'isolation thermique souple (52).

8. Accessoire de nettoyage à la vapeur prêt-à-porter selon l'une quelconque des revendications 6 et 7 dans lequel l'au moins un conduit de vapeur (26) est lié à la seconde couche d'isolation thermique souple (54).

9. Accessoire de nettoyage à la vapeur prêt-à-porter selon l'une quelconque des revendications précédentes dans lequel l'élément de nettoyage souple (32) est un ou plusieurs des suivants : un tissu de nettoyage, un tampon à récurer, des fibres de brosse ou une éponge.

10. Accessoire de nettoyage à la vapeur prêt-à-porter selon l'une quelconque des revendications précédentes dans lequel l'élément de nettoyage souple (32) est monté de manière amovible sur l'au moins une couche d'isolation thermique souple (30, 50).

11. Accessoire de nettoyage à la vapeur prêt-à-porter selon l'une quelconque des revendications précédentes dans lequel l'au moins un conduit de vapeur (26) comprend un tube flexible comportant une section transversale en forme de D.

12. Accessoire de nettoyage à la vapeur prêt-à-porter selon l'une quelconque des revendications précédentes dans lequel l'au moins une sortie de vapeur (26) est montée sur une surface de l'au moins un conduit de vapeur qui fait face à l'élément de nettoyage souple (32).

13. Accessoire de nettoyage à la vapeur prêt-à-porter selon l'une quelconque des revendications précédentes dans lequel le dispositif de retenue (46) comprend une poche comportant un matériau à mailles pour faire circuler l'air près de la main d'un utilisateur.

14. Accessoire de nettoyage à la vapeur prêt-à-porter selon l'une quelconque des revendications précédentes dans lequel l'au moins une couche d'isolation thermique souple (30, 50) comprend une paroi périphérique faisant saillie vers le dispositif de retenue souple pour sensiblement entourer la main d'un utilisateur.

15. Dispositif de nettoyage à la vapeur (10) comprenant ;
un accessoire de nettoyage à la vapeur prêt-à-porter (20) selon les revendications 1 à 14 ; et
un dispositif de génération de vapeur (14) couplé à l'adaptateur.
